# EUROPEAN PATENT APPLICATION

(11) **EP 4 556 889 A1**
(43) Date of publication of application: **21.05.2025**
(21) Application number: 23210037.0
(22) Date of filing: 15.11.2023
(51) Int. Cl.: G01N 21/23, G01N 21/25, G01N 21/31, G01N 21/87, B07C 5/342, G01N 33/38, G01N 33/40, G01N 21/85

(54) **DETECTION OF EMERALDS**

(71) Applicant: TOMRA Sorting GmbH, 56218 Mülheim-Kärlich (DE)
(72) Inventor: SHIBISTOVA, Alina, 22589 Hamburg (DE); DEHLER, Markus, 22559 Hamburg (DE)
(74) Representative: AWA Sweden AB

(57) **Abstract**

The present invention relates to a system (1) for detecting emeralds in an object (O) in a detection region (DR). The system (1) comprises a laser arrangement (2) comprising a first laser (2A) configured to emit radiation having a wavelength within a first interval and a second laser (2B) configured to emit radiation having a wavelength within a second interval, a light redirecting arrangement (4) configured to redirect optical radiation originating from the laser arrangement (2) towards a portion of the detection region (DR), and a spectroscopy system (10) configured to receive and analyze radiation eminating from said object (O) when in said detection region (DR), wherein the spectroscopy system (10) comprises a first detector (10A) configured to detect and measure a first intensity of at least one wavelength band within the first interval and a second detector (10B) configured to detect and measure a second intensity of at least one wavelength band within the second interval, wherein the spectroscopy system (10) is further configured to compare the first intensity with the second intensity, and, if the difference is larger than a predetermined threshold value, classifying the object (O) as comprising emerald.

## Description

### Technical field

The present invention relates to a system and a method for detecting emeralds in objects.

### Background

Throughout a wide range of industries identification, detection, classification and sorting of various objects are frequently required and desired.

In its simplest form, manual identification of objects by a person may be employed to advantage when a limited number of objects are to be identified, sorted and classified. The person in question may then, based on his/her knowledge identify and classify the objects concerned. This type of manual identification is however monotonous and prone to errors. Also, the experience level of the operator will significantly influence the results of the operation performed by the operator. Moreover, manual identification of the above kind suffers from low identification speeds.

In industry, identification, sorting and classification of bulk objects is therefore often performed by machines where the bulk objects are supplied in some form of a continuous object stream. Such machines are generally faster than an operator and can operate for longer periods of time, hence offering an enhanced overall throughput. Machines of this kind are for instance used in the mining industry to sort crushed ore based on its composition. Further, machines of this kind are used to identify valuable parts, such as gems, in crushed rock or ore.

Machines of the above kind generally has some form of sensor that is used for identifying the objects of interest. Generally, a color camera is used to image gem containing rock and to identify gemstones included in the rock or present in the form of unenclosed crystals. Gemstones may thus typically be identified based on their color. For instance, color-based identification of emeralds has become common practice in the mining industry.

Generally, detection of unenclosed crystals using a color camera allows for an efficient identification of gems, such as emeralds. However, when the gemstones of interest are intergrown or otherwise included in the rock it has proven difficult to achieve a satisfactory identification. Further, identification of intergrown or included gemstones has proven difficult both for light and dark colored rock. The problem of identification becomes even more pronounced when the rock at hand is coarsely grained.

In order to address identification problems related to intergrown or included gemstones it has been suggested to increase the sensitivity of the settings used to identify the gemstones of interest. However, when the sensitivity is increased this will generally result in a significant over-identification which in turn will require a secondary identification process to identify the actual gemstones. In other words, the rock particles or pieces identified using the high sensitivity settings will have to be examined once more in order to discard particles or pieces that do actually not include any gemstones. The secondary identification will consequently have to rely on a different identification principle, such as manual identification. Such manual secondary identification is both costly, time consuming and prone to further identifications errors which in turn may result in loss of valuable gemstones.

### Summary

To achieve at least one of the above objects and also other objects that will be evident from the following description, a system having the features defined in claim 1 is provided according to the present inventive concept. Preferred embodiments will be evident from the dependent claims.

According to a first aspect of the present inventive concept, a system for detecting emeralds in an object in a detection region is provided, the system comprising: a laser arrangement comprising a first laser configured to emit radiation having a wavelength within an interval from and including 810 nm to and including 850 nm and a second laser configured to emit radiation having a wavelength within an interval from and including 510 nm to and including 550 nm; a light redirecting arrangement configured to redirect optical radiation originating from the laser arrangement towards a portion of the detection region, such that the irradiated portion moves from a first end of said detection region to a second end of said detection region; and a spectroscopy system configured to receive and analyze radiation eminating from said object when in said detection region, wherein the spectroscopy system comprises a first detector configured to detect and measure a first intensity of at least one wavelength band within the interval of 810 nm to 850 nm and a second detector configured to detect and measure a second intensity of at least one wavelength band within the interval of 510 nm to 550 nm; wherein the spectroscopy system is further configured to compare the first intensity with the second intensity, and, if the difference is larger than a predetermined threshold value, classifying the object as comprising emerald.

Within the context of the present disclosure, *"Emerald"* is to be understood as green colored mineral crystal material of beryl (Be₃Al₂(SiO₃)₆).

Due to emerald exhibiting a low green spectrum absorption and high IR absorption, it provides a specific signature facilitating detection thereof. Thus, the present system provides for a more robust and effective way of detecting emerald in objects, especially for emeralds that are at least partially includes within the object.

It is to be understood that comparing a first intensity with a second intensity may be performed in a variety of ways, e.g. by dividing the first intensity with the second intensity, or vice versa. Thus, a predetermined threshold value may, e.g., be a quotient of 6, 5, 4, 3, or 2, or 1/6, 1/5, 1/4, 1/3 or 1/2.

The first laser may additionally or alternatively be configured to emit radiation having a wavelength within an interval from and including 815 nm to and including 845 nm, or 820 nm to 840 nm, or 825 nm to 835 nm. The second laser may additionally or alternatively be configured to emit radiation having a wavelength within an interval from and including 515 nm to and including 545 nm, or 520 nm to 540 nm, or 525 nm to 535 nm.

The first and/or the second detector may be configured to detect and measure more than one intensity each within the respective intervals.

The first laser and the second laser may be linearly polarized, and the spectroscopy system may optionally be provided with one or more polarization filter or polarizors, e.g. one or two polarizors arranged after each other and acting on all or a portion of the radiation received by the spectrometer and/or one polarization filter is arranged in front of each one of the detectors, which polarization filter or polarizer may be configured to fully or substantially block radiation with polarization of the first laser and/or the second laser.

The first laser and the second laser are linearly polarized, wherein the spectroscopy system is provided with at least one polarization filter in the optical path before one or both of the first and the second detector, and wherein the at least one polarization filter is configured to substantially block radiation with a polarization coinciding with the polarization of said one or both of the first laser and the second laser.

According to one example, one polarization filter is arranged in the optical path before the first detector, and is configured to substantially block radiation with a polarization coinciding with the polarization of at least said first laser. Additionally or alternatively, one polarization filter is arranged in the optical path before the second detector, and is configured to substantially block radiation with a polarization coinciding with the polarization of at least said second laser
According to one example one, several or all of the above discussed polarizors or polarizing filters, which each is configured to block a predetermined polarization, is configured such that it blocks at least 90 % of the intensity of the radiation having this predetermined polarization.

According to one example, said one or two polarization filters are arranged in a part of the optical path common both said first and second detector, or each one of said one or two polarization filters extends across the optical path of both the first and second detector.

The radiation emitted by the first laser and the radiation emitted by the second laser may be coinciding.

The laser arrangement may comprise any number of further lasers. For example, the laser arrangement may comprise a third laser, wherein the third laser is configured to emit red spectrum radiation. Any subset of lasers may arranged such that their respective radiation is coinciding.

The object may be stationary in the detection region. In other words, the object may be stationary in the detection region while being scanned. To this end, the light redirecting arrangement may be further configured so as to move the irradiated portion through the detection region in a raster pattern.

It is to be understood that more than one object may be scanned at a time.

Alternatively, the object may be moving through the detection region while being scanned. To this end, the system may further comprise a means for transporting the object through the detection region, wherein the means may be a conveyor or a chute for sliding or frefalling the object.

Hereby, a continuous stream of objects may be scanned for detection of emeralds therein. Thus, a more efficient system is provided.

Optical radiation originating from the laser arrangement may be redirected to the detection region in a variety of ways. For example, the light redirecting arrangement may comprise a polygon mirror configured to rotate around a rotation axis and comprising a plurality of reflective surfaces arranged one after another around the rotation axis, wherein each reflective surface may be configured to redirect optical radiation originating from the laser arrangement such that the irradiated portion moves from a first end of said detection region to a second end of the detection region once per reflective surface and revolution of the polygon mirror.

The polygon mirror may further be configured to precess, i.e. move in a way such that the rotation axis is moving. For example, the rotation axis may oscillate in a plane, such that optical radiation originating from the laser arrangement may be redirected to the detection region in a raster pattern.

Additionally or alternatively, the light redirecting arrangement may comprise a tilting mirror. The radiation received and analyzed by the spectroscopy system may be directed thereto along an optical path via the polygon mirror or the tilting mirror.

According to a second aspect of the present inventive concept, a method for detecting emeralds in an object in a detection region by a system according to the first aspect is provided, the method comprising: redirecting, by the light redirecting arrangement, radiation originating from the laser arrangement towards a portion of the detection region, such that the irradiated portion moves from a first end of said detection region to a second end of said detection region; receiving and analyzing, by the spectroscopy system, radiation eminating from said object when in said detection region, comprising detecting and measuring, by the first detector of the spectroscopy system, a first intensity of at least one wavelength band within the interval of 810 nm to 850 nm and detecting and measuring, by the second detector of the spectroscopy system, a second intensity of at least one wavelength band within the interval of 510 nm to 550 nm; and comparing the first intensity with the second intensity, and, if the difference is larger than a predetermined threshold value, classifying the object as comprising emerald.

To avoid undue repitition, any technical effect or benefit discussed with regards to the first aspect of the present inventive concept is applicable to the second aspect of the present inventive concept. Furthermore, any variant discussed with regards to the first aspect of the present inventive concept may be applicable to the second aspect of the present inventive concept.

Specifically, the first laser and the second laser may be linearly polarized, wherein the step of receiving and analyzing further may comprise transmitting radiation with polarization which is perpendicular to the polarization of the first laser and/or the second laser by a polarization filter of the spectroscopy system.

The method may further comprise transporting the object through the detection region by a conveyor or a chute for sliding or frefalling said object through said detection region.

### Brief Description of the Drawings

The invention will in the following be described in more detail with reference to the enclosed drawings, wherein:
Fig. 1 shows the absorption over the UV-VIS-NIR spectra for emerald, displaying the difference in absorption between ordinary rays (o rays) and extraordinary rays (e ray);
Fig. 2 is a schematic view of a system for detecting emeralds in accordance with the inventive concept;
Fig. 3 is a schematic side view of a system for detecting emeralds in accordance with the inventive concept.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

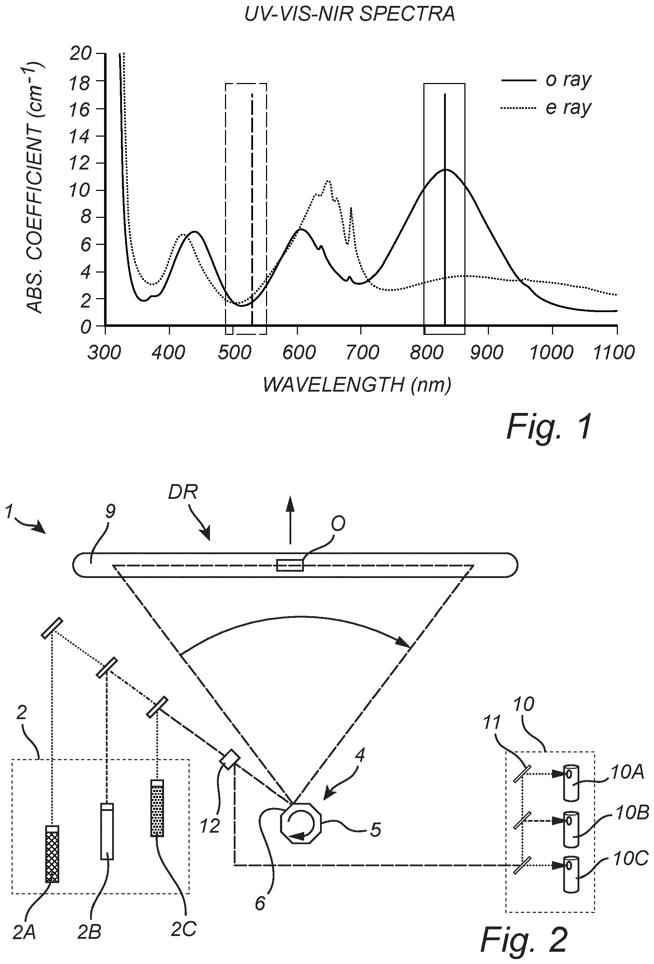

In Fig. 1, a graph of absorption over the UV-VIS-NIR spectra for emerald is provided. Since emerald is birefractive, incident unpolarized light will split into ordinary rays and extraordinary rays. In Fig. 1, the absorption spectrum of ordinary rays (o rays) is displayed in a solid line, whereas the absorption spectrum of extraordinary rays (e rays) is displayed in a dotted line. A first important characteristic of the spectra is that there is a low total absorption in the interval of about 490 nm to about 550. Furthermore, as is visible, there is a peak of total absorption within the interval of about 800 nm to about 875 nm, the contribution to which mainly comes from the o ray spectrum. In contrast, the difference in absorption between o rays and e rays within the interval of about 490 nm to about 550 nm is very small.

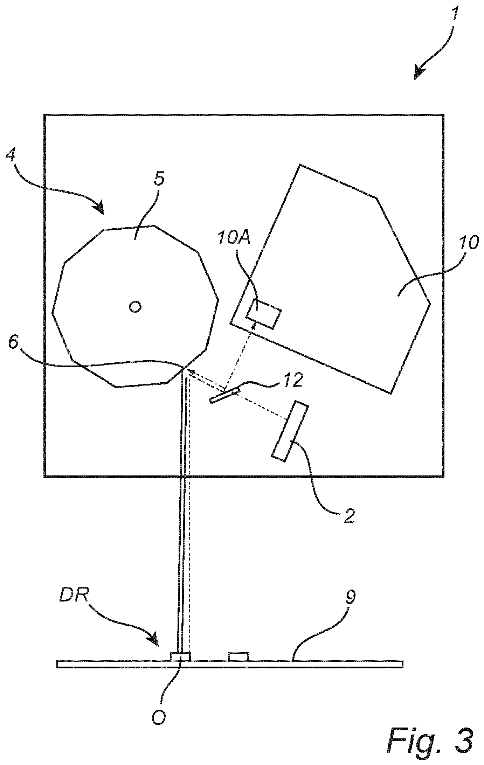

The spectroscopy system 1, which will be explained further below in relation to Fig. 2 and Fig. 3, of the first and second aspect of the present inventive concept is thus configured to detect and measure an intensity of at least one wavelength band within the interval of 810 nm to 850 nm and at least one wavelength band within the interval of 510 nm to 550 nm or 490 nm to 550 nm as shown in Fig 1. Here, the respective wavelength bands are at approximately 530 nm and 830 nm. However, it is to be understood that the respective wavelength bands may be chosen anywhere in the indicated intervals. Furthermore, the respective wavelength bands are In Fig. 1 indicated as being narrow, i.e. in the order of a few nanometers. However, it is to be understood that the wavelength bands may be of any width within the respective intervals. For example, any wavelength band may have a width of 20 nm, or 15 nm, or 10 nm, or 9 nm, or 8 nm, or 7 nm, or 6 nm, or 5 nm, or 4 nm, or 3 nm, or 2 nm, or 1 nm.

In Fig. 2, a system 1 for detecting emeralds in accordance with the present inventive concept is shown. The system 1 is configured for detecting emeralds in an object O in a detection region DR. An object O may be any kind of object. An object O may, e.g., be a rock. Here, a single object O is shown. However, any number of objects O may be present.

The system 1 comprises a laser arrangement 2. The laser arrangement comprising a first laser 2A a second laser 2B. The first laser 2A is configured to emit radiation having a wavelength within an interval from and including 810 nm to and including 850 nm, and the second laser 2B is configured to emit radiation having a wavelength within an interval from and including 510 nm to and including 550 nm. The first laser 2A may additionally or alternatively be configured to emit radiation having a wavelength within an interval from and including 815 nm to and including 845 nm, or 820 nm to 840 nm, or 825 nm to 835 nm. The second laser 2B may additionally or alternatively be configured to emit radiation having a wavelength within an interval from and including 515 nm to and including 545 nm, or 520 nm to 540 nm, or 525 nm to 535 nm. Here, the laser arrangement 2 further comprises a third laser 2C. The third laser may be configured to emit radiation having a wavelength within any interval. For example, the third laser 2C may be configured to emit radiation having a wavelength within the same interval as the first laser 2A or the second laser 2B. Alternatively, the third laser 2C may be configured to emit radiation having a wavelength within an interval from and including 550 nm to 600 nm, or 560 to 590 nm, or 570 to 580 nm.

The radiation emitted from the first laser 2A and/or the second laser 2B and/or the third laser 2C may be polarized. For example, any laser 2A, 2B, 2C may be lineraly polarized.

The system 1 further comprises a light redirecting arrangement 4 configured to redirect optical radiation originating from the laser arrangement 2 towards a portion of the detection region DR, such that the irradiated portion moves from a first end of said detection region DR to a second end of said detection region DR. Here, the light redirecting arrangement 4 comprises a polygon mirror 5. The polygon mirror 5 is configured to rotate around a rotation axis, as is indicated by the curved arrow in Fig. 2. The polygon mirror 5 comprises a plurality of reflective surfaces 6 arranged one after another around the rotation axis. Each reflective surface 6 is configured to redirect optical radiation originating from the laser arrangement 2 such that the irradiated portion moves from a first end of said detection region to a second end of the detection region DR once per reflective surface 6 and revolution of the polygon mirror 5.

Here, the radiation emitted from the lasers 2A, 2B, 2C are coinciding. That is, the radiation emitted from the lasers 2A, 2B, 2C are coinciding when incident upon the polygon mirror 5 and any reflective surface 6 thereof. To this end, the system 1 comprises three semi-transparent mirrors, 8 arranged so as to collect and align radiation emitted from the lasers 2A, 2B, 2C, such that they are coinciding. However, it is to be understood that coincidation of radiation emitted from any number of lasers may be facilitated in a number of ways. For example, semi-transparent mirrors 8, one or more beam splitters or one or more dichroic mirrors may be used to transmit and redirect the radiation of interest in the desired direction.

The radiation redirected by the light redirecting arrangement 4 is redirected towards the detection region DR. Here, the detection region DR at least partially covers a surface 9 arranged to support the object O. However, the detection region DR may alternatively be defined as a plane or volume of free space. This is e.g. true for variants where an object 0 is frefalling through the detection region DR. It is to be understood that the surface 9 may be formed or provided in a wide variety of ways. For example, the surface 9 may be that of a stationary table configured for supporting an object O during scanning. Alternatively, the surface 9 may be that of a means for transporting the object O through the detection region DR. Thus, the surface 9 may be that of a conveyor or a chute. For example, the surface 9 is provided by a conveying surface of a conveyor transporting the object through the detection region in a direction indicated by the straight arrow in Fig. 1. Hereby, the light redirecting arrangement 4 is configured to redirect optical radiation originating from the laser arrangement 2 towards a portion of the detection region DR, which portion moves along a direction perpendicular to the direction of transportation of the object O.

The system 1 further comprises a spectroscopy system 10 configured to receive and analyze radiation eminating from the object O when in the detection region DR. To this end, the spectroscopy system 10 comprises a first detector 10A configured to detect and measure a first intensity of at least one wavelength band within the interval of 810 nm to 850 nm. Further, the spectroscopy system 10 comprises a second detector 10B configured to detect and measure a second intensity of at least one wavelength band within the interval of 510 nm to 550 nm. Here, the spectroscopy system 10 further comprises a third detector 10C. The third detector 10C may e.g. be configured to detect and measure a third intensity of at least one wavelength band within the interval of the first detector 10A or the second detector 10B. Alternatively, the third detector 10C may be configured to detect and measure a third intensity of at least one wavelength band within the interval from and including 550 nm to 600 nm. It is to be understood that the spectroscopy system 10 may comprise any nymber of detectors. The spectroscopy system 10 may comprise more than one detector configured to detect and measure the intensity of a wavelength band within the same wavelength interval. For example, the spectroscopy system 10 may comprise more than one of the first detector 10A. Furthermore, the first 10A and/or the second 10B and/or the third 10C detector may be configured to detect and measure more than one intensity each within the respective intervals.

In order to split the incoming radiation to the respective detectors, a number of altrnative components may be used. For example, three dichroic mirrors 11 may be arranged such that radiation of the prescribed wavelength reaches the detector configured for analyzing such wavelength.

The spectroscopy system 10 is configured to compare the first intensity with the second intensity. This comparison may be performed in a variety of ways, e.g. by dividing the first intensity with the second intensity, or vice versa. If the difference is larger than a predetermined threshold value, which may, e.g., be a quotient of 6, 5, 4, 3, or 2, or 1/6, 1/5, 1/4, 1/3 or ½, the object O is classified as comprising emerald. Classifying the object as comprising emerald is to be understood as the object O being classified as comprising emerald material in any form. The emerald material may be detected as one continuous object, or a plurality of objects. As such, the present inventive concept may further be used for mapping of emerald material included in the object O. For example, mapping of emerald material may comprise determining a percentage of emerald to host material of said object O.

The detectors 10A, 10B and 10C may be configured to detect and measure ordinary rays, such that the first intensity is the intensity of ordinary rays of at least one wavelength band within the interval of 810 nm to 850 nm, and such that the second intensity is the intensity of ordinary rays of at least one wavelength band within the interval of 510 nm to 550 nm. To this end, the first laser and the second laser may be linearly polarized, wherein the spectroscopy system may be provided with one or more polarizors configured to fully or substantially block radiation with polarization of the first laser and/or the second laser.

The radiation eminating from the object O may reach the spectroscopy system 10 in a variety of ways. For example, the radiation may be directed thereto along an optical path via the polygon mirror 5. To this end, the system 1 further comprises a semi-transparent mirror 12. The system 1 may comprise more than one semi-transparent mirror 12. This may also be facilitated by one or more beam splitters. Alternatively, this may be facilitated by one or more dichroic mirrors.

The arrangement of the mirror 12 is further illustrated in Fig. 3, where the system 1 is shown from a perspective different than the on in Fig. 2. Here, optical radiation emitted from the laser arrangement 2 is transmitted through the mirror 12, reaches a reflective surface 6 of the polygon mirror, where it is reflected towards the object O in the detection region DR. Part of the radiation is then eminated back to the reflective surface 6, where it is reflected towards the mirror 12, where it is reflected towards the spectroscopy system 10, which system 10 receives and analyzes the radiation.

Thus, the system 1 is configured to detect emeralds in the object O in the detection region DR by redirecting, by the light redirecting arrangement 4, radiation originating from the laser arrangement 2 towards a portion of the detection region DR, such that the irradiated portion moves from a first end of said detection region DR to a second end of said detection region DR, and receiving and analyzing, by the spectroscopy system 10, radiation eminating from the object O, wherein receiving and analyzing comprises detecting and measuring, by the first detector 10A of the spectroscopy system 10, a first intensity of at least one wavelength band within the interval of 810 nm to 850 nm and detecting and measuring, by the second detector 10B of the spectroscopy system 10, a second intensity of at least one wavelength band within the interval of 510 nm to 550 nm, and comparing the first intensity with the second intensity, and, if the difference is larger than a predetermined threshold value, classifying the object O as comprising emerald.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A system for detecting emeralds in an object in a detection region, the system comprising:
a laser arrangement comprising a first laser configured to emit radiation having a wavelength within an interval from and including 810 nm to and including 850 nm and a second laser configured to emit radiation having a wavelength within an interval from and including 510 nm to and including 550 nm;
a light redirecting arrangement configured to redirect optical radiation originating from the laser arrangement towards a portion of the detection region, such that the irradiated portion moves from a first end of said detection region to a second end of said detection region; and
a spectroscopy system configured to receive and analyze radiation eminating from said object when in said detection region, wherein the spectroscopy system comprises a first detector configured to detect and measure a first intensity of at least one wavelength band within the interval of 810 nm to 850 nm and a second detector configured to detect and measure a second intensity of at least one wavelength band within the interval of 510 nm to 550 nm;
wherein the spectroscopy system is further configured to compare the first intensity with the second intensity, and, if the difference is larger than a predetermined threshold value, classifying the object as comprising emerald.

2. The system according to claim 1 or 2, wherein the first laser and the second laser are linearly polarized, wherein the spectroscopy system is provided with at least one polarization filter in the optical path before one or both of the first and the second detector, and wherein the at least one polarization filter is configured to substantially block radiation with a polarization coinciding with the polarization of said one or both of the first laser and the second laser.

3. The system according to any preceding claim, wherein the radiation emitted by the first laser and the radiation emitted by the second laser are coinciding.

4. The system according to any preceding claim, further comprising a means for transporting said object through said detection region, wherein said means is a conveyor or a chute for sliding or frefalling said object.

5. The system according to any preceding claim, wherein the light redirecting arrangement comprises a polygon mirror configured to rotate around a rotation axis and comprising a plurality of reflective surfaces arranged one after another around the rotation axis, wherein each reflective surface is configured to redirect optical radiation originating from the laser arrangement such that the irradiated portion moves from a first end of said detection region to a second end of said detection region once per reflective surface and revolution of the polygon mirror.

6. The system according to any preceding claim, wherein the light redirecting arrangement comprising a tilting mirror.

7. The system according to claim 6 or 7, wherein the radiation received and analyzed by the spectroscopy system is directed thereto along an optical path via the polygon mirror or the tilting mirror.

8. A method for detecting emeralds in an object in a detection region by a system according to any one of the preceding claims, the method comprising:
redirecting, by the light redirecting arrangement, radiation originating from the laser arrangement towards a portion of the detection region, such that the irradiated portion moves from a first end of said detection region to a second end of said detection region;
receiving and analyzing, by the spectroscopy system, radiation eminating from said object when in said detection region, comprising detecting and measuring, by the first detector of the spectroscopy system, a first intensity of at least one wavelength band within the interval of 810 nm to 850 nm and detecting and measuring, by the second detector of the spectroscopy system, a second intensity of at least one wavelength band within the interval of 510 nm to 550 nm; and
comparing the first intensity with the second intensity, and, if the difference is larger than a predetermined threshold value, classifying the object as comprising emerald.

9. The method according to claim 9, wherein detecting and measuring a first intensity comprises detecting and measuring intensity of ordinary rays of at least one wavelength band within the interval of 810 nm to 850 nm, and detecting and measuring a second intensity comprises detecting and measuring intensity of ordinary rays of at least one wavelength band within the interval of 510 nm to 550 nm, such that comparing the first intensity with the second intensity comprises comparing the intensities of ordinary rays within the respective intervals.

10. The method according to claim 9 or 10, wherein the first laser and the second laser are linearly polarized, wherein the step of receiving and analyzing further comprises transmitting radiation with polarization which is perpendicular to the polarization of the first laser and/or the second laser by a polarization filter of the spectroscopy system.

11. The method according to any one of claims 9 to 11, wherein the radiation emitted by the first laser and the radiation emitted by the second laser are coinciding.

12. The method according to any one of claims 9 to 12, further comprising transporting said object through said detection region by a conveyor or a chute for sliding or frefalling said object through said detection region.

13. The method according to any one of claims 9 to 13, wherein the light redirecting arrangement comprises a polygon mirror such that redirecting radiation originating from the laser arrangement is performed by rotating the polygon mirror around a rotation axis thereof such that each reflective surface redirects radiation so as to move the irradiated portion from a first end of said detection region to a second end of said detection region once per reflective surface and revolution of the polygon mirror.

14. The method according to claim 14, wherein the step of receiving and analyzing further comprises directing radiation eminating from said object when in siad detection region to the spectroscopy system along an optical path via the polygon mirror.
